# FASCICULE DE BREVET EUROPEEN

(11) **EP 0 941 252 B1**
(45) Date de publication et mention de la délivrance du brevet: **05.12.2007**
(21) Numéro de dépôt: 97912262.9
(22) Date de dépôt: 27.10.1997
(51) Int. Cl.: C07K 16/18, C12N 15/13, A61K 39/395

(54) **FRAGMENTS D'ANTICORPS A CHAINE UNIQUE ANTI-P53 ET UTILISATIONS**
ANTI-P53 SPEZIFISCHE EINKETTIGE-ANTIKÖRPERFRAGMENTE UND DEREN VERWENDUNG
ANTI-P53 SINGLE-CHAIN ANTIBODY FRAGMENTS AND THEIR USES

(30) Priorité: 29.10.1996 FR 9613176
(43) Date de publication de la demande: 15.09.1999
(73) Titulaire: Aventis Pharma S.A., 92160 Antony (FR)
(72) Inventeur: BRACCO, Laurent, F-75013 Paris (FR); DEBUSSCHE, Laurent, F-91200 Athis Mons (FR)
(86) Numéro de dépôt international: PCT/FR1997/001921
(87) Numéro de publication internationale: WO 1998/018825

(56) Documents cités:
- WO-A-94/12202
- WO-A-96/30512
- WO-A-97/04092
- ABARZUA P. ET AL.,: "Microinjection of monoclonal antibody Pab421 into human sw480 colorectal carcinoma cells restores the transcription activation function to mutant p53" CANCER RESERACH, vol. 55, - 15 août 1995 pages 3490-3494, XP002035503
- HUPP T R ET AL: "REGULATION OF THE SPECIFIC DNA BINDING FUNCTION OF P53" CELL, vol. 71, 27 novembre 1992, pages 875-886, XP000619238
- HALAZONETIS T D ET AL: "WILD-TYPE P53 ADOPTS A "MUTANT"-LIKE CONFORMATION WHEN BOUND TO DNA" EMBO JOURNAL, vol. 12, no. 3, 1993, pages 1021-1028, XP000619249
- JANNOT C.B. & HYNES N.E.: "Characterization of scFv-421, a single-chain antibody targeted to p53" BIOCHEMICAL AND BIOPHYSICAL RESEARCH COMMUNICATIONS, vol. 230, - 13 janvier 1997 pages 242-246, XP002035502
- CARON DE FROMENTEL C. ET AL: "Restoration of transcriptional activity of p53 mutants in humn tumour cells by intracellular expression of anti-p53 single chain Fv fragments" ONCOGENE, vol. 18, 1999, pages 551-557,

## Description

La présente invention concerne un procédé pour restaurer dans des cellules présentant une protéine p53 mutée, dépourvue ou diminuée de sa fonction de facteur transcriptionnel, une activité de transactivation p53-dépendante. Plus particulièrement, le procédé de l'invention repose sur l'utilisation d'anticorps simple chaine capables de lier spécifiquement la protéine p53 mutée. Elle concerne également de nouvelles molécules capables de lier spécifiquement et efficacement les protéines p53, et permettant en outre de restaurer une activité p53 dans des cellules tumorales, ainsi que les acides nucléiques codant pour ces molécules et les vecteurs les contenant. Ce procédé et les molécules de l'invention sont utilisables in vitro ou ex vivo pour étudier le mécanisme d'action de p53 et de ses formes mutées ou pour purifier les protéines p53. Ils présentent également des utilisations in vivo, notamment dans des approches thérapeutiques de restauration d'activité p53 dans des contextes pathologiques tels que notamment les cancers.

La protéine p53 sauvage intervient dans la régulation du cycle cellulaire et dans le maintien de l'intégrité du génome de la cellule. Cette protéine, dont la fonction principale est d'être un activateur de la transcription de certains gènes, est susceptible, par un processus non encore bien défini, de bloquer la cellule en phase G1 du cycle cellulaire lors de l'apparition de mutations au cours de la réplication du génome, et d'enclencher un certains nombre de processus de réparation de l'ADN. De plus, en cas de mauvais fonctionnement de ces processus de réparation ou en cas d'apparition d'événements mutationnels trop nombreux pour être corrigés, cette protéine est capable d'induire le phénomène de mort cellulaire programmée, appelé apoptose. De cette façon, la protéine p53 agit comme un supresseur de tumeur, en éliminant les cellules anormalement différenciées ou dont le génome a été endommagé.

Cette principale fonction de la p53 dépend de sa fonction de facteur de transcription, soit en d'autre termes de sa double capacité à reconnaître des séquences spécifiques au niveau de l'ADN génomique et à recruter la machinerie générale de transcription.

La protéine p53 comporte 393 acides aminés, qui définissent 5 domaines fonctionnels :
- le domaine activateur de la transcription, constitué par les acides aminés 1 à 73, capable de lier certains facteurs de la machinerie générale de transcription comme la protéine TBP. Ce domaine est aussi le siège d'un certain nombre de modifications post-traductionnelles. Il est également le siège d'interactions nombreuses de la protéine p53 avec de nombreuses autres protéines et notamment avec la protéine cellulaire mdm2
ou la protéine EBNA5 du virus d'Epstein-Barr (EBV), capables de bloquer la fonction de la protéine sauvage. De plus, ce domaine possède des séquences d'acides aminés dites PEST de susceptibilité à la dégradation protéolytique.
- le domaine de liaison à l'ADN, localisé entre les acides aminés 73 et 315. La conformation de ce domaine central de p53 régule la reconnaissance de séquences d'ADN spécifiques de la protéine p53. Ce domaine est le siège de deux types d'altérations affectant la fonction de la protéine sauvage :
   (i) l'interaction avec des protéines bloquant la fonction de la p53 comme l'antigène 'grand T' du virus SV40 ou les protéines virales E6 des virus HPV16 et HPV18 capables de provoquer sa dégradation par le système de l'ubiquitine. Cette dernière interaction ne peut se faire qu'en présence de la protéine cellulaire E6ap (enzyme E3 de la cascade de l'ubiquitinilation).
   (ii) les mutations ponctuelles qui affectent la fonction de la p53 et dont la quasi-totalité sont localisées dans cette région.
- le signal de localisation nucléaire, constitué des acides aminés 315 à 325, indispensable au bon adressage de la protéine dans le compartiment où elle va exercer sa principale fonction.
- le domaine d'oligomérisation, constitué des acides aminés 325 à 355. Cette région 325 à 355 forme une structure de type; feuillet β (326-334)-coude (335-336)-hélice α (337-355). Les altérations de fonctions localisées dans cette région sont essentiellement dues à l'interaction de la protéine sauvage avec les différentes formes mutantes qui peuvent conduire à des effets variables sur la fonction de la protéine sauvage.
- le domaine de régulation, constitué des acides aminés 365 à 393, qui est le siège d'un certain nombre de modifications post-traductionnelles (glycosylations, phosphorylations, fixation d'ARN,...) qui modulent la fonction de la protéine p53 de façon positive ou négative. Ce domaine joue un rôle extrèmement important dans la modulation de l'activité de la protéine sauvage.

Le fonctionnement de la protéine p53 peut être perturbé de différentes façons.
- blocage de sa fonction par un certain nombre de facteurs comme par exemple l'antigène 'grand T' du virus SV40, la protéine EBNA5 du virus d'Epstein-Barr, ou la protéine cellulaire mdm2.
- déstabilisation de la protéine par augmentation de sa susceptibilité à la protéolyse, notamment par interaction avec la protéine E6 des virus du papillome humain HPV16 et HPV18, qui favorise l'entrée de la p53 dans le cycle d'ubiquitinilation. Dans ce cas l'interaction entre ces deux protéines ne peut se faire que par la fixation préalable d'une protéine cellulaire, la protéine E6ap dont le site de fixation est mal connu.
- mutations ponctuelles au niveau du gène de la p53.
- délétion d'un ou des deux allèles de la p53

Les deux derniers types de modifications sont retrouvés dans environ 50% des différents types de cancer. A cet égard, les mutations du gène de la p53 répertoriées dans les cellules cancéreuses touchent une très grande partie du gène codant pour cette protéine, et ont pour résultats des modifications variables du fonctionnement de cette protéine. On peut cependant noter que ces mutations sont en grande majorité localisées dans la partie centrale de la protéine p53 dont on sait qu'elle est la région de contact avec les séquences génomiques spécifiques de la protéine p53. Ceci explique pourquoi la plupart des mutants de la protéine p53 ont comme principale caractéristique de ne plus pouvoir se fixer aux séquences d'ADN que reconnait la protéine sauvage et ainsi de ne plus pouvoir exercer leur rôle de facteur de transcription. Par ailleurs, certains mutants semblent avoir acquis de nouvelles fonctions telles que l'activation de certains gènes au niveau transcriptionnel.

On regroupe actuellement l'ensemble de ces modifications dans trois catégories:
- les mutants dits faibles, dont le produit est une protéine non-fonctionnelle, qui, dans le cas de mutation sur un seul des deux allèles, n'affecte pas le fonctionnement de la protéine sauvage codée par l'autre allèle. Les principaux représentants de cette catégorie sont les mutants H273 et W248, ce dernier étant spécifique du syndrome familial de Li-Fraumeni d'hypersensibilité aux affections cancéreuses.
- les mutants dominant-négatifs, dont le produit est une protéine non-fonctionnelle, qui, dans le cas de mutation sur un seul des deux allèles et par intéraction avec la protéine sauvage, est capable de bloquer le fonctionnement de celle-ci par formation d'oligomères mixtes non-actifs qui ne peuvent plus se fixer aux séquences d'ADN spécifiques de la protéine sauvage. Le principal représentant de cette catégorie est le mutant G281.
- les mutants dominant-oncogéniques, dont le produit est une protéine qui est capable d'une part de bloquer la fonction de la protéine sauvage comme les mutants de la catégorie précédente, et d'autre part, de favoriser par des mécanismes mal connus le développement tumoral, présentant ainsi un gain de fonction. Le principal représentant de cette catégorie est le mutant H175.

Compte tenu de ses propriétés anti-tumorales et apoptotiques et de son implication dans nombreuses pathologies de type hyperprolifératives, le gène p53 sauvage a été utilisé dans des approches de thérapie génique et cellulaire. Il a en particulier été proposé de traiter certaines pathologies hyperprolifératives, et notament des cancers, par administration in vivo du gène p53 sauvage, par restauration des fonctions de p53. L'administration peut être réalisée préférentiellement par des vecteurs viraux et notamment adénoviraux (WO94/24297) ou rétroviraux (WO94/06910). Il a ainsi été montré que l'introduction d'un acide nucléique codant pour la protéine p53 sauvage permettait de restaurer partiellement une régulation normale de la croissance cellulaire (Roth et al., Nature Medicine 2 (1996) 985). Des stratégies alternatives basées sur l'emploi de molécules chimères ayant des propriétés de type p53 ont également été développées (PCT/FR96/01111).

Une autre approche pour restaurer les fonctions de la protéine p53 sauvage est basée sur la réversion des protéines mutées endogènes vers un phénotype sauvage, c'est-à-dire présentant les propriétés suppresseur de tumeur et apoptotiques de la p53 sauvage. Cette approche découle de la mise en évidence que les pertes de fonction des mutants de p53 sont dues à un changement conformationnel de la protéine induit par la/les mutations. A cet égard, la demande WO94/12202 montre qu'un anticorps monoclonal particulier, désigné pAb421, dirigé contre la protéine p53, est capable de restaurer à une certaine classe de mutants fréquemment représentés dans les cancers humains la fonction de liaison à l'ADN in vitro. L'utilisation de ce type de composés présente cependant des inconvénients importants, liés notamment à la quantité élevée d'anticorps nécessaire (et donc aux problèmes de production/purification associés) et à leur faible pénétration intracellulaire.

La présente demande décrit une approche plus performante pour restaurer les propriétés sauvage d'un mutant de la protéine p53. La présente demande décrit en particulier la construction de ligands particulièrement spécifiques de la protéine p53, ayant- des propriétés avantageuses de restauration des fonctions p53 sauvage. Plus particulièrement, la présente demande décrit la construction d'anticorps simple chaine (ScFv) spécifiques de la protéine p53, et notamment la molécule 11D3. La présente demande montre en outre que les ScFv sont capables de reconnaitre p53, d'être exprimés efficacement au sein d'une cellule tumorale et de réactiver une partie de la fonction transactivatrice d'une certaine classe de mutants de p53.

Par rapport aux méthodes de l'art antérieur, cette molécule présente des avantages importants et notamment la possibilité d'être exprimée in situ dans une cellule tumorale, en quantités importantes: Les résultats présentés ci-après sont d'autant plus inattendus que des pertes d'affinité avaient souvent été observées entre un anticorps classique et un ScFv. En outre, la demanderesse a montré qu'il est possible d'exprimer les ScFv dans les compartiments intracellulaires appropriés, permettant d'obtenir une activité biologique optimale.

Le document WO96/30512 est relatif à un système d'expression conditionnelle qui fait appel à des molécules chimériques bispécifiques comprenant (A) un premier domaine capable de lier sélectivement une séquence définie d'ADN et (B) un deuxième domaine détecteur capable de lier spécifiquement un transactivateur ou un complexe transactivateur, ce deuxième domaine peut être un ScFv dirigé contre p53. La restauration des fonctions transactivatrices de la protéine p53 avec une construction comprenant seulement un scFv ou une séquence codant pour un scFv et dépourvue d'élément A, n'est pas décrite dans ce document.

Le document CancerResearch, Vol 55, Aug 15, 1995, pp 3490 - 3494, enseigne que l'anticorps monoclonal mAb PA421est capable de restaurer la fonction d'activation de la transcription de certaines formes mutées de p53, cependant ce document ne fournit aucun information sur les propriétés d'éventuels ScFv dérivés de cet anticorps monoclonal.

Un premier objet de l'invention réside donc dans un procédé pour restaurer dans des cellules présentant une protéine p53 mutée une activité de transactivation p53-dépendante comprenant l'introduction dans ladite cellule d'un anticorps simple chaine capable de lier spécifiquement la protéine p53 mutée. Avantageusement, le procédé de l'invention comprend l'introduction dans la cellule d'un acide nucléique comprenant une séquence codant pour ledit anticorps simple chaine sous le contrôle d'un promoteur fonctionnel dans la cellule. Un autre aspect de l'invention concerne l'utilisation d'un anticorps simple chaine chaine capable de lier spécifiquement une protéine p53 mutée pour modifier la conformation de ladite protéine. L'invention concerne également l'utilisation d'un anticorps simple chaine capable de lier spécifiquement une protéine p53 mutée pour la préparation d'une composition pharmaceutique destinée au traitement des désordres hyperprolifératifs dans lesquels une protéine p53 mutée est impliquée, ainsi que l'utilisation d'un acide nucléique codant pour un anticorps simple chaine capable de lier spécifiquement une protéine p53 mutée pour la préparation d'une composition pharmaceutique destinée au traitement des désordres hyperprolifératifs dans lesquels une protéine p53 mutée est impliquée.

Le procédé de l'invention repose donc en partie sur la construction, la vectorisation et l'introduction dans des cellules d'anticorps simple chaine capables de lier spécifiquement une protéine p53 mutée. Les anticorps simple-chaine (ScFv) sont constitués essentiellement d'une région VH liée à une région VL par un bras. La construction de ScFv et des séquences d'acides nucléiques codant pour de tels anticorps modifiés a été décrite par exemple dans le brevet US4,946,778 ou dans les demandes WO94/02610, WO94/29446.

La présente demande décrit plus particulièrement la création d'une banque d'hybridomes produisant des anticorps dirigés contre p53, et la construction, à partir de cette banque, de ScFv correspondants. Elle décrit également le clonage des acides nucléiques correspondants dans des vecteurs d'expression et leur transfert dans des cellules. Elle montre également que ce transfert permet, in vivo, de restaurer efficacement l'activité de liaison à l'ADN de mutants de p53, ainsi que leur activité de transactivateur.

Plus particulièrement, le procédé de l'invention met en oeuvre des ScFv capables de lier spécifiquement un épitope présent dans la région C-terminale de p53, qui porte le domaine d'oligomérisation et le domaine de régulation. A cet égard, la présente demande décrit également un test permettant de sélectionner les ScFv possédant cette propriété, par technique ELISA.

Encore plus préférentiellement, les ScFv utilisés dans le procédé de l'invention sont capables de lier spécifiquement un épitope présent dans la région C-terminale de p53 comprise entre les résidus 320-393. A cet égard, la demande décrit à titre d'exemple particulier la construction et l'expression du ScFv ScFv421 de séquence SEQ ID n° 1 et du 11 D3 de séquence SEQ ID n° 2.

Le procédé de l'invention est applicable généralement aux protéines p53 mutées ayant perdu, totalement ou partiellement, la capacité de lier l'ADN, et le procédé de l'invention permet de restaurer cette capacité. Plus particulièrement, le procédé de l'invention est applicable aux protéines p53 mutées ayant perdu, totalement ou partiellement, la fonction de facteur transcriptionnel de p53 et il permet de restaurer cette fonction. Le niveau de restauration peut être total ou partiel. Avantageusement, il est suffisant pour permettre au mutant d'exercer une fonction de suppresseur de tumeur, par bloquage du cycle cellulaire et/ou par induction d'apoptose. Le procédé de l'invention permet donc de restaurer, au moins partiellement, une activité suppresseur de tumeur dans des cellules présentant des protéines p53 mutées endogènes dépourvues de cette activité. Avantageusement, il s'agit de protéines mutées présentes dans les cellules tumorales. Comme indiqué ci-avant, différentes formes mutées de la protéine p53 ont été mises en évidence dans les cellules tumorales. On peut citer par exemple les protéines p53H273, p53W248 et p53G281. Les exemples présentés ci-après montrent en particulier que le procédé de l'invention permet de modifier la conformation, et les propriétés biologiques de ces mutants, in vitro et in vivo. En particulier, ces exemples démontrent que les ScFv 421 et 11D3 sont capables de restaurer aux mutants 273 et 248 la capacité de lier spécifiquement l'ADN, et d'induire la transactivation p53 dépendante.

Le procédé de l'invention peut être mis en oeuvre in vitro, ex vivo ou in vivo. In vitro ou ex vivo le procédé et les molécules de l'invention peuvent permettre par exemple d'étudier le mécanisme d'action de p53 et de ses formes mutées. En outre, les molécules de l'invention peuvent être utilisées pour la détection ou la purification de protéines p53, par exemple par couplage sur un support, mise en contact avec une solution contenant des protéines p53, suivie éventuellement d'une révélation des complexes formés ou d'une élution. In vivo, notamment chez l'homme, ils peuvent permettre, dans des contextes pathologiques tels que les désordres hyperprolifératifs dans lesquels une déficience en activité p53 est observée, de restaurer cette fonction. A cet égard, il peut être utilisé en association avec d'autres approches évoquées ci-avant (introduction d'un gène p53 sauvage) ou également en association avec une chimiothérapie (WO96/22101). Toujours in vivo, le procédé et les molécules de l'invention sont utilisables chez l'animal, par exemple pour déterminer les niveaux d'expression des ScFv, et évaluer la possibilité d'une approche thérapeutique humaine.

Avantageusement, la cellule présentant une protéine p53 mutée est une cellule tumorale mammifère. A cet égard, on peut citer plus particulièrement les cellules des cancers du poumon (notamment non à petites cellules), du coton, du foie, du cerveau, tête et cou et, plus généralement, tout cancer dans lesquels une forme mutée de la protéine p53 est observée. Avantageusement, il s'agit d'une cellule tumorale humaine dans laquelle un mutant p53H273, p53W248 et/ou p53G281 est observé (poumon, colon, cerveau, tête et cou, foie). L'applicabilité du procédé de l'invention à une cellule particulière peut être déterminée aisément selon la méthodologie suivante : La cellule est tout d'abord caractérisée pour la présence d'une protéine p53 mutée. Cette protéine est ensuite caractérisée pour déterminer la nature de la mutation. S'il s'agit d'une mutation connue, et notamment répertoriée ci-dessus, la cellule peut être considérée comme susceptible de traitement par le procédé de l'invention. S'il s'agit d'une mutation non-répertoriée, différentes approches sont possibles. La protéine mutée peut tout d'abord être isolée (ou synthétisée artificiellement) et testée comme décrit dans les exemples pour son comportement in vitro et in vivo en présence de ScFv. Ceci permet d'identifier le ScFv approprié pour restaurer les fonctions déficientes de cette protéine. Une autre approche consiste à tester directement les ScFv sur une culture de cellules, pour déterminer l'efficacité biologique des ScFv.

Pour la mise en oeuvre du procédé de l'invention, le ScFv est avantageusement introduit dans la cellule, in vitro, ex vivo ou in vivo sous forme d'un vecteur portant un acide nucléique codant pour ledit ScFv sous controle d'un promoteur fonctionnel dans ladite cellule.

Le promoteur est avantageusement choisi parmi les promoteurs fonctionnels dans les cellules mammifères, de préférence humaines. Plus préférentiellement, il s'agit d'un promoteur permettant l'expression d'un acide nucléique dans une cellule hyperproliférative (cancéreuse, resténose, etc). A cet égard, différents promoteurs peuvent être utilisés. Il peut s'agir par exemple du propre promoteur du gène p53. Il peut également s'agir de régions d'origine différente (responsables de l'expression d'autres protéines, ou même synthétiques). Il peut ainsi s'agir de tout promoteur ou séquence dérivée stimulant ou réprimant la transcription d'un gène de façon spécifique ou non, inductible ou non, forte ou faible. On peut citer notamment les séquences promotrices de gènes eucaryotes ou viraux. Par exemple, il peut s'agir de séquences promotrices issues du génome de la cellule cible. Parmi les promoteurs eucaryotes, on peut utiliser en particulier de promoteurs ubiquitaires (promoteur des gènes HPRT, PGK, a-actine, tubuline, etc), de promoteurs des filaments intermédiaires (promoteur des gènes GFAP, desmine, vimentine, neurofilaments, kératine, etc), de promoteurs de gènes thérapeutiques (par exemple le promoteur des gènes MDR, CFTR, Facteur VIII, ApoAl, etc), de promoteurs spécifiques de tissus (promoteur du gène pyruvate kinase, villine, protéine intestinale de liaison des acides gras, a-actine du muscle lisse, etc) ou encore de promoteurs répondant à un stimulus (récepteur des hormones stéroïdes, récepteur de l'acide rétinoïque, etc). De même, il peut s'agir de séquences promotrices issues du génome d'un virus, tel que par exemple les promoteurs des gènes E1A et MLP d'adénovirus, le promoteur précoce du CMV, ou encore le promoteur du LTR du RSV, etc. En outre, ces régions promotrices peuvent être modifiées par addition de séquences d'activation, de régulation, ou permettant une expression tissu-spécifique ou majoritaire.

Comme indiqué ci-avant, la présente demande décrit également de nouvelles molécules ayant des propriétés de liaison et de reversion de protéines p53 mutées, particulièrement avantageuses. L'invention décrit plus précisément la construction, la vectorisation et le transfert dans des cellules de ScFv particuliers (11D3, 421). La séquence nucléique et peptidique de ces ScFv est indiquée SEQ ID n° 1 et 2. Les exemples qui suivent montrent la capacité particulièrement avantageuse de ces molécules (i) de lier spécifiquement les protéines p53 mutées, dans la région C-terminale, (ii) de rendre à ces protéines mutées la capacité de lier l'ADN, et (iii) de rendre à ces protéines la capacité d'activer la transcription. Les exemples montrent en outre que ces molécules sont correctement exprimées dans les cellules tumorales, ce qui permet leur utilisation avantageuse dans des contextes de désordres hyperprolifératifs. Par ailleurs, les propriétés des ScFv de l'invention peuvent également être améliorées. Notamment, il est connu que l'affinité des ScFv est influencée par les régions CDR (soulignées sur la séquence) et peut être améliorée par des expériences routinières de mutagénèse/sélection. Ainsi, la mutagénèse sur des anticorps a été par exemple décrite par Marks et coll. (Bio/Technology, 10, 779-783, 1992) et par Winter G. et Milstein C. (Nature, 349,293-299, 1991). Les technologies décrites dans ces références peuvent être appliquées à la préparation de variants des ScFv ayant une affinité modifiée selon l'invention. La sélection peut ensuite être faite dans les conditions décrites dans les exemples.

L'invention a donc en outre pour objet la molécule 11D3 dont la séquence peptidique est présentée SEQ ID n°2, ainsi que tout variant présentant une modification dans les régions CDR, conservant la capacité de lier les protéines p53. La modification peut consituer en une délétion, une substitution ou une insertion d'un ou plusieurs résidus dans les régions CDR. Avantageusement, la modification porte sur moins de 10 résidus.

La présente invention a également pour objet tout acide nucléique codant pour le ScFv 11D3 ou un variant tel que défini ci-avant.

L'acide nucléique selon l'invention peut être un acide ribonucléique (ARN) ou désoxyribonucléique (ADN). Avantageusement, il s'agit d'un ADN complémentaire (ADNc). Il peut être d'origine humaine, animale, virale, synthétique ou semi-synthétique. Il peut être obtenu de différentes manières et notamment par synthèse chimique en utilisant les séquences présentées dans la demande et par exemple un synthétiseur d'acides nucléiques. Il peut également être obtenu par criblage de banques au moyen de sondes spécifiques, notamment telles que décrites dans la demande. Il peut encore être obtenu par des techniques mixtes incluant la modification chimique (élongation, délétion, substitution, etc) de séquences criblées à partir de banques. D'une manière générale, les acides nucléiques de l'invention peuvent être préparés selon toute technique connue de l'homme du métier (voir notamment les technologies décrites dans les brevets US4,946,778 et WO94/02610. Une stratégie classique de construction d'acides nucléiques codant pour un ScFv est la suivante : Les cDNA codant pour les régions VH et VL sont obtenus à partir de l'hybridome produisant l'anticorps anti-p53 choisi. Pour cela, les ARN totaux de l'hybridome sont extraits et soumis à une réaction de transcription inverse en utilisant des hexamères random comme amorces. L'utilisation de ce type d'amorce permet d'éviter l'emploi d'amorces spécifiques des immunoglobulines. Les clones d'ADNc obtenus ont une longueur suffisante pour cloner les régions V. Lorsqu'ils représentent une trop faible fraction des cDNA totaux présents, une réaction préalable d'amplification peut être réalisée pour produire suffisamment de DNA pour le clonage. Pour cela, les ADNc codant pour les régions VH et VL sont amplifiés séparément. Les amorces utilisées sont des oligonucléotides hybridant au niveau des extrémités opposées des régions variables de chaque chaine (H et L). Le produit d'amplification utilisant les amorces spécifiques des chaines lourdes et le produit d'amplification utilisant les amorces spécifiques des chaines légères sont ensuite purifiés. Après purification, les ADNc codant pour les régions VH et VL de l'anticorps sont assemblés en une chaine unique au moyen d'un bras nucléotidique (L). Le bras nucléotidique a été construit de telle sorte que l'une des extrémités se lie à l'extrémité 3' de l'ADNc codant pour la région VH et l'autre à l'extrémité 5' de l'ADNc codant pour la région VL. La séquence du bras code pour le peptide (G4S)3. La séquence assemblée, de 700 pb environ, contient sous forme d'un fragment Ncol-Notl, l'enchainement VH-L-VL dont les séquences sont représentées SEQ ID n° 1 et 2 par exemple.

Préférentiellement, l'acide nucléique selon l'invention est un ADNc ou un ARN.

L'acide nucléique selon l'invention est avantageusement choisi parmi :
(a) tout ou partie de la séquence SEQ ID n° 2 ou de son brin complémentaire,
(b) toute séquence hybridant avec les séquences (a) et codant pour un ScFv capable de lier spécifiquement la protéine p53, de préférence au niveau de la région C-terminale,
(c) les variants de (a) et (b) résultant de la dégénérescence du code génétique.

La présente invention concerne également toute cassette d'expression comprenant un acide nucléique tel que défini ci-avant, un promoteur permettant son expression et un signal de terminaison de la transcription.

Dans le procédé de l'invention, l'acide est avantageusement introduit dans les cellules au moyen d'un vecteur d'administration, qui permet d'améliorer (i) l'efficacité de la pénétration cellulaire, (ii) le ciblage et/ou (iii) la stabilité extra- et intracellulaires.

Dans un mode de mise en oeuvre particulièrement préféré de la présente invention l'acide nucléique est incorporé dans un vecteur qui peut être d'origine chimique (liposome, nanoparticule, complexe peptidique, lipides ou polymères cationiques, etc) virale (rétrovirus, Adénovirus, virus de l'herpès, AAV, virus de la vaccine, etc) ou plasmidique.

L'utilisation de vecteurs viraux repose sur les propriétés naturelles de transfection des virus. Il est ainsi possible d'utiliser par exemple les adénovirus, les herpès virus, les rétrovirus et les virus adéno associés. Ces vecteurs s'avèrent particulièrement performants sur le plan de la transfection.

En particulier, la capacité des adénovirus et des rétrovirus à infecter les cellules tumorales en font des vecteurs de choix dans le cadre de l'invention. A cet égard, dans un mode préféré de mise en oeuvre de l'invention, l'acide nucléique est introduit sous forme d'un vecteur rétroviral, c'est-à-dire d'un rétrovirus recombinant défectif dont le génome comprend un acide nucléique codant pour un ScFv tel que défini ci-avant. Dans un autre mode préféré de mise en oeuvre de l'invention, l'acide nucléique est introduit sous forme d'un vecteur adénoviral, c'est-à-dire d'un adénovirus recombinant défectif dont le génome comprend un acide nucléique codant pour un ScFv tel que défini ci-avant.

Le vecteur selon l'invention peut également être un agent non viral capable de promouvoir le transfert et l'expression d'acides nucléiques dans des cellules eucaryotes. Les vecteurs chimiques ou biochimiques, synthétiques ou naturels, représentent une alternative intéressante aux virus naturels en particulier pour des raisons de commodité, de sécurité et également par l'absence de limite théorique en ce qui concerne la taille de l'ADN à transfecter. Ces vecteurs synthétiques ont deux fonctions principales, compacter l'acide nucléique à transfecter et promouvoir sa fixation cellulaire ainsi que son passage à travers la membrane plasmique et, le cas échéant, les deux membranes nucléaires. Pour pallier à la nature polyanionique des acides nucléiques, les vecteurs non viraux possèdent tous des charges polycationiques. Dans un procédé particulier selon l'invention, le vecteur est un vecteur chimique ou biochimique.

L'invention concerne également toute composition comprenant au moins un acide nucléique tel que défini ci-avant.

Elle concerne également toute composition comprenant au moins un vecteur tel que défini ci-avant.

Elle concerne aussi toute composition comprenant au moins un ScFv tel que défini ci-avant.

Elle concerne également des compositions comprenant un acide nucléique ou un vecteur tel que défini ci-avant et un acide nucléique ou un vecteur codant pour la p53 sauvage, pour une utilisation combinée simultanée ou espacée dans le temps.

En raison de leurs propriétés antiprolifératives, les compositions pharmaceutiques selon l'invention sont tout particulièrement adaptées pour le traitement des désordres hyperprolifératifs, tels que notamment les cancers et la resténose. La présente invention fournit ainsi une méthode particulièrement efficace pour la destruction de cellules, notamment de cellules hyperprolifératives.

Elle peut être utilisée in vitro ou ex vivo. Dans ce cas, elle consiste essentiellement à incuber les cellules en présence d'un ou plusieurs acides nucléiques (ou d'un vecteur, ou cassette ou directement du ScFv). Des doses de 0.01 à 1000 µg de vecteur pour 10⁶ cellules, ou une MOI de 0,1 à 1000 pour un vecteur viral peuvent être utilisées.

In vivo, elle consiste à administrer à l'organisme une quantité active d'un vecteur (ou d'une cassette) selon l'invention, de préférence directement au niveau du site à traiter (tumeur notamment). A cet égard, l'invention a également pour objet une méthode de destruction de cellules hyperprolifératives comprenant la mise en contact desdites cellules ou d'une partie d'entre-elles avec un acide nucléique tel que défini ci-avant. Pour une utilisation in vivo, l'acide nucléique ou le vecteur utilisé dans la présente invention peut être formulé en vue d'administrations par voie topique, orale, parentérale, intranasale, intraveineuse, intramusculaire, sous-cutanée, intraoculaire, transdermique, etc. De préférence, l'acide nucléique ou le vecteur est utilisé sous une forme injectable. Il peut donc être mélangé à tout véhicule pharmaceutiquement acceptable pour une formulation injectable, notamment pour une injection directe au niveau du site à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Une injection directe de l'acide nucléique dans la tumeur du patient est intéressante car elle permet de concentrer l'effet thérapeutique au niveau des tissus affectés. Les doses d'acide nucléique utilisées peuvent être adaptées en fonction de différents paramètres, et notamment en fonction du gène, du vecteur, du mode d'administration utilisé, de la pathologie concernée ou encore de la durée du traitement recherchée. Avantageusement, les doses administrées in vivo sont comprises entre 10⁶ et 10¹⁰ pfu pour un vecteur viral tel qu'un adénovirus. Par ailleurs, des administrations répétées peuvent également être envisagées.

Toujours in vivo, le procédé et les molécules de l'invention peuvent être utilisées pour étudier les mécanismes d'action de p53, et pour déterminer le potentiel des ScFv sur des modèles animaux.

La présente invention est avantageusement utilisée in vivo pour la destruction de cellules en hyperprolifération (i.e. en prolifération anormale). Elle est ainsi applicable à la destruction des cellules tumorales ou des cellules de muscle lisse de la paroi vasculaire (resténose). Elle est tout particulièrement appropriée au traitement des cancers dans lesquels un mutant de p53 est observé. A titre d'exemple, on peut citer les adénocarcinomes du colon, les cancers de la thyroïde, les carcinomes du poumon, les leucémies myéloïdes, les cancers colorectaux, les cancers du sein, les cancers du poumon, les cancers gastriques, les cancers de l'oesophage, les lymphômes B, les cancers ovariens, les cancers de la vessie, les glioblastomes, les hépatocarcinomes, les cancers des os, de la peau, du pancréas ou encore les cancers du rein et de la prostate, les cancers de l'oesophage, les cancers du larynx, les cancers tête et cou, les cancers ano-génitaux HPV positifs, les cancers du nasopharynx EBV positifs, les cancers dans lesquels la protéine cellulaire mdm2 est surexprimée, etc.

La présente invention est décrite plus en détail dans les exemples qui suivent, qui doivent être considérés comme illustratifs et non limitatifs.

### Légende des Figures

Figure 1 : Stratégie de criblage des anticorps
Figure 2 : Mise en évidence de la capacité des anticorps à induire un retard sur gel de p53.
Figure 3 : Mise en évidence de la capacité des anticorps à induire un retard sur gel de p53H273.
Figure 4 : Mise en évidence par ELISA de l'association des ScFv avec p53 sauvage. Ronds pleins : lgG 11 D3 (1 µg/ml au départ); Ronds vides : lgG 421 (1 µg/ml au départ); Carrés : sérum polyclonal biotinylé (1 µg/ml au départ); Triangles pleins : ScFv 11 D3-myc (au ½ au départ); Triangles vides : ScFv 421-myc (au ½ au départ); Losanges : ScFv irrelevant (anti-CD3, au ½ au départ).
Figure 5 : Mise en évidence des retards sur gel induits par les ScFv
Figure 6 : Restauration de l'activité de liaison à l'ADN à des mutants de p53.
Figure 7 : Expression du ScFv421 dans les cellules H1299
Figure 8 : Restauration de l'activité transcriptionnelle du mutant H273 dans la lignée H358.
Figure 9 : Restauration de l'activité transcriptionnelle du mutant H273 endogène dans la lignée HT29.

### Exemples

### EXEMPLE 1 : Obtention et criblage de l'anticorps 11D3

Cet exemple décrit la préparation, l'obtention et la sélection d'anticorps monoclonaux dirigés spécifiquement contre la protéine p53, capables d'activer la fonction de liaison à l'ADN des formes mutées de p53.

### 1.1. Obtention des protéines utilisées lors de l'immunisation des souris et le criblage des hybridomes.

La protéine p53 sauvage et differentes protéines p53 H273, p53 W248, p53 G281 correspondant à des mutations de la p53 sauvage fréquemment retrouvées dans des cellules tumorales ont été produites dans des cellules d'insectes *Spodoptera frugiperda* Sf9 infectées par un baculovirus recombinant et purifiées par affinité sur un gel d'agarose sur lequel a été couplé l'anticorps polyclonal PAb421 (Leveillard et al. , EMBO J. 15, 1615-1623 (1996)). Les protéines correspondant aux fragments 1-320 et 73-320 de la protéine p53 sauvage ont été produites également dans des cellules d'insectes *Spodoptera frugiperda* Sf9 infectées par un baculovirus recombinant en suivant les instructions données par la Société Invitrogen. Les ADN complémentaires inserés dans le plasmide de transfert pBlueBacIII ont été générés par des techniques classiques de l'ADN recombinant décrites par exemple par Sambrook et al. (Sambrook, Fritsch & Maniatis : Molecular cloning, a laboratory manual, 2nd edition , 1989, Ed. Cold Spring Harbor Laboratory).

### 1.2. Obtention et criblage des hybridomes

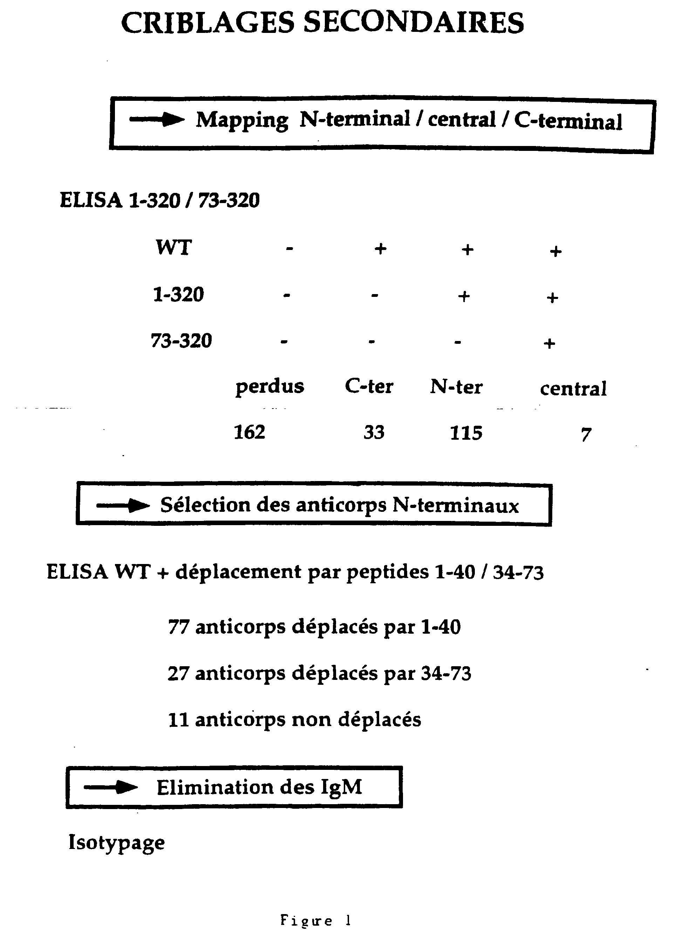

Les souris ont été immunisées avec un mélange équimolaire des trois protéines mutantes de p53 décrites ci-avant et les hybridomes ont été obtenus en suivant les protocoles opératoires décrits par Harlow et Lane (Harlow & Lane : Antibodies, a laboratory manual, 1988, Ed. Cold Spring Harbor Laboratory). La sélection des hybridomes produisant des anticorps monoclonaux dirigés contre les protéines mutantes de p53 décrites ci-avant a été réalisée par la méthode de capture de l'anticorps produit dans le milieu de culture de l'hybridome dans des puits de plaques 96 puits en PVC dans lesquels avait été au préalable immobilisée une quantité de 1 microgramme du mélange équimolaire des trois protéines mutantes de p53 décrites ci-avant en suivant les protocoles opératoires décrits par Harlow et Lane (Harlow & Lane : Antibodies, a laboratory manual, 1988, Ed. Cold Spring Harbor Laboratory). Ce premier criblage a permis de sélectionner 317 hybridomes positifs. Après deux semaines d'amplification des hybridomes, les surnageants des hybridomes amplifiés ont été dans un premier temps réévalués par la méthode (méthode 1) de capture de l'anticorps mentionée ci-avant puis classés en utilisant trois méthodes de criblage identiques dans le principe à celle mentionée ci-avant sauf que la nature des protéines immobilisées était différente : dans les puits étaient immobilisés soit (méthode 2) la protéine p53 sauvage purifiée, soit (méthode 3) un extrait protéique de cellules Sf9 produisant le fragment 1-320 de la p53 sauvage, soit (méthode 4) un extrait protéique de cellules Sf9 produisant le fragment 73-393 de la p53 sauvage. Les extraits protéiques ont été obtenus par lyse par congélations/décongélations dans un tampon phosphate des cellules Sf9 puis ultracentifugation des débris cellulaires. Sur les 317 surnageants d'hybridomes amplifiés, 162 ne répondaient plus dans la méthode 1. Les 155 restant étaient tous positifs dans la méthode 2. parmi ceux-ci, 33 (groupe A) étaient négatifs dans les méthodes 3 et 4, 115 (Groupe B) étaient positifs dans la méthode 3 et négatifs dans la méthode 4, et enfin 7 (groupe C) étaient positifs dans les deux méthodes. Les surnageants du groupe B correspondent aux surnageants contenant un anticorps dont l'épitope se situe dans les 73 premiers acides aminés de p53. 77 surnagenants de ce groupe se sont avérés négatifs dans la méthode 2 quand ils étaient préincubés avec le peptide (1 mg/ml) correspondant à la séquence des 40 premiers acides aminés de p53. Un isotypage des anticorps du groupe A, des 38 anticorps du groupe B restant après élimination des 77 mentionés ci-avant et de ceux du groupe C nous a permis d'éliminer les IgM. Ces résultats sont récapitulés dans la Figure 1.

42 anticorps ont ensuite été testés pour leur capacité à induire un supershift sur un complexe p53/DNA. Après purification sur protéine A/Sepharose, les anticorps ont été quantifiés. Des experiences de retard sur gel ont été réalisées en incubant 30ng de protéine p53 de type sauvage purifiée avec une sonde d'ADN marquée au 32P représentant une séquence de fixation spécifique de p53. 300ng des divers anticorps ont ensuite été ajoutés. Les complexes ont été résolus sur gel d'acrylamide.

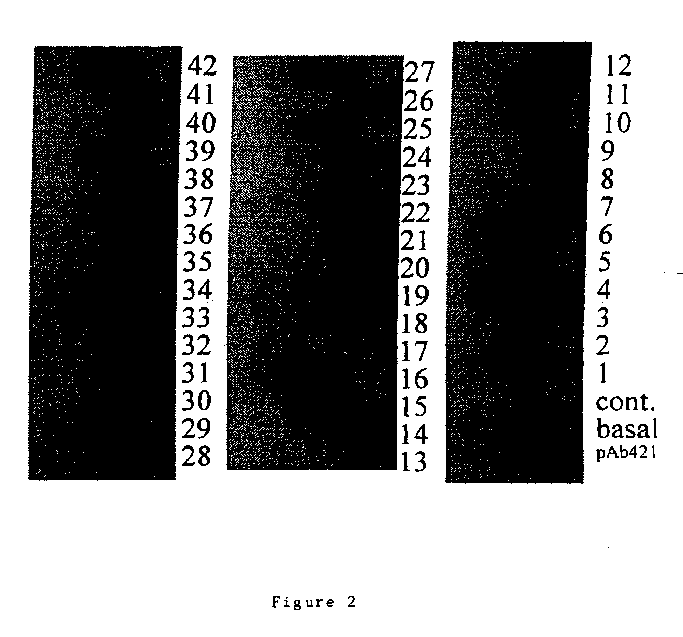

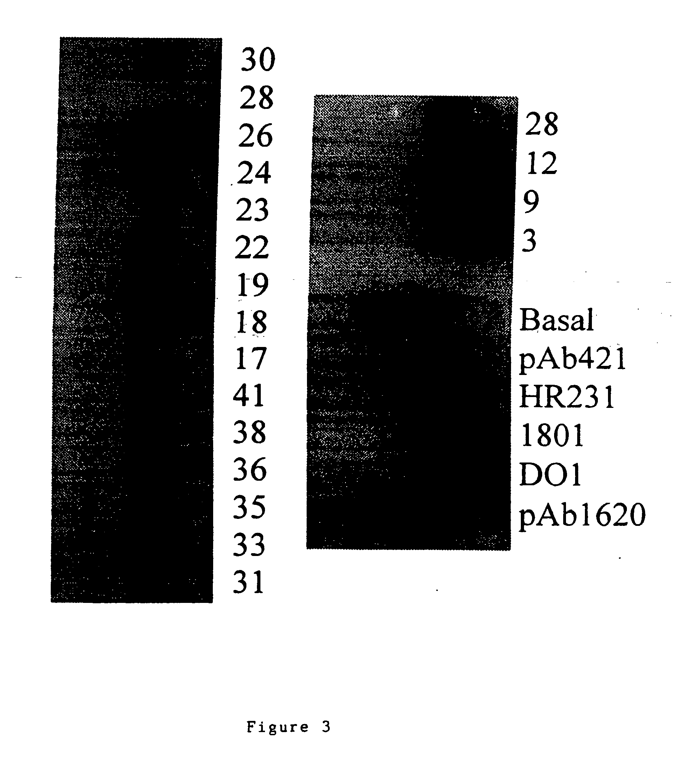

Les résultats de la Figure 2 montrent que 27 de ces anticorps étaient capables d'induire un supershift. 19 parmi ces 27 ont été téstés en substituant le mutant His273 à la protéine p53. sauvage dans la même experience de retard sur gel.(Figure 3). Ces 19 anticorps ont tous donné des resultats positifs. L'anticorps n°26 présentait un retard plus prononcé que les autres. Cet anticorps a été désigné 11 D3 et utilisé dans la suite des expériences.

### EXEMPLE 2 : Obtention des ScFvs 421 et D3M

Les ScFvs ont été obtenus à partir des hybridomes par des techniques classiques de biologie moléculaire basées sur des PCR à l'aide d'amorces dégénérées spécifiques des régions VH et VL. Le ScFv dérivé de l'anticorps 11D3 a été désigné D3M. Sa séquence est représentée sur la SEQ ID n°2. La séquence du ScFv421 est représentée sur la SEQ ID n°1.

### EXEMPLE 3 : Construction de vecteurs d'expression des ScFv

Cet exemple décrit la construction de vecteurs utilisables pour le transfert des acides nucléiques de l'invention in vitro ou in vivo.

### 3.1. Construction de vecteurs plasmidiques

Pour la construction de vecteurs plasmidiques, 2 types de vecteurs ont été utilisés.
- Le vecteur pSV2, décrit dans DNA Cloning, A practical approach Vol.2, D.M. Glover (Ed) IRL Press, Oxford, Washington DC, 1985. Ce vecteur est un vecteur d'expression eucaryote. Les acides nucléiques codant pour les ScFv ont été insérés dans ce vecteur sous forme de fragments Hpal-EcoRV. Ils sont ainsi placés sous le contrôle du promoteur de l'enhancer du virus SV40. Toutes les constructions décrites dans l'exemple 2 ont été introduites dans ce vecteur pour être testées dans les différents systèmes d'évaluation in vitro et in vivo.
- Le vecteur pCDNA3 (Invitrogen). Il s'agit également d'un vecteur d'expression eucaryote. Les acides nucléiques codant pour les ScFv de l'invention sont ainsi placés, dans ce vecteur, sous le controle du promoteur précoce du CMV. Toutes les constructions décrites dans l'exemple 2 ont été introduites dans ce vecteur sous forme d'un fragment Hind III / Not I.

### 3.2. Construction de vecteurs viraux

Selon un mode particulier, l'invention réside dans la construction et l'utilisation de vecteurs viraux permettant le transfert et l'expression in vivo des acides nucléiques tels que définis ci-avant.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprenent les ITR, une séquence permettant l'encapsidation et un acide nucléique selon l'invention. Encore plus préférentiellement, dans le génome des adénovirus de l'invention, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697, WO96/22378) et L5 (WO95/02697). Selon un mode préféré de mise en oeuvre, l'adénovirus selon l'invention comprend une délétion dans les régions E1 et E4. Selon un autre mode de réalisation préféré, il comprend une délétion dans région E1 au niveau de laquelle sont insérés la région E4 et l'acide nucléique de l'invention (WO96/13596). Dans les virus de l'invention, la délétion dans la région E1 s'étend préférentiellement des nucléotides 455 à 3329 sur la séquence de l'adénovirus Ad5.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN d'intérêt. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914, WO95/02697 et WO96/22378.

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (lTR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant une séquence nucléique de l'invention d'intérêt bordée de deux régions répétées inversées (ITR) d'AAV, et d'un plasmide portant les gènes d'encapsidation (gènes rep et cap) d'AAV. Une lignée cellulaire utilisable est par exemple la lignée 293. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants selon l'invention comportant un acide nucléique selon l'invention, un plasmide comportant notamment les LTR, la séquence d'encapsidation et ledit acide nucléique est construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

Pour la mise en oeuvre de la présente invention, il est tout particulièrement avantageux d'utiliser un adénovirus ou un rétrovirus recombinant défectif. Ces vecteurs possèdent en effet des propriétés particulièrement intéressantes pour le transfert de gènes dans les cellules tumorales.

### 3.3. Vecteurs chimiques

Parmi les vecteurs synthétiques développés, on préfère utiliser dans le cadre de l'invention les polymères cationiques de type polylysine, (LKLK)n, (LKKL)n (WO95/21931), polyéthylène immine (WO96/02655) et DEAE dextran ou encore les lipides cationiques ou lipofectants. Ils possèdent la propriété de condenser l'ADN et de promouvoir son association avec la membrane cellulaire. Parmi ces derniers, on peut citer les lipopolyamines (lipofectamine, transfectam, WO95/18863, WO96/17823) différents lipides cationiques ou neutres (DOTMA, DOGS, DOPE, etc) ainsi que des peptides d'origine nucléaire (WO96/25508). En outre, le concept de la transfection ciblée a été développé, médiée par un récepteur, qui met à profit le principe de condenser l'ADN grâce au polymère cationique tout en dirigeant la fixation du complexe à la membrane grâce à un couplage chimique entre le polymère cationique et le ligand d'un récepteur membranaire, présent à la surface du type cellulaire que l'on veut greffer. Le ciblage du récepteur à la transferrine, à l'insuline ou du récepteur des asialoglycoprotéines des hépatocytes a ainsi été décrit. La préparation d'un composition selon l'invention utilisant un tel vecteur chimique est réalisée selon toute technique connue de l'homme du métier, généralement par simple mise en contact des différents composants.

### EXEMPLE 4 : Les scFvs reconnaissent p53

L'association des ScFvs avec p53 a été vérifiée en test ELISA.

Un tag myc a été fusionné aux scFvs pour permettre leur detection. Les ScFvs 421, 11 D3 (D3M) et un ScFv controle (anti-CD3) ont été produits à partir de périplasmes de bactéries exprimant ces différents ScFvs.

Des plaques ELISA coatées à l'aide de p53 purifiée sont incubées avec différentes dilutions de l'IgG 11 D3, l'IgG 421, un sérum polyclonal biotynilé anti p53, le scFv 11 D3, le ScFv 421 et le ScFv anti-CD3.

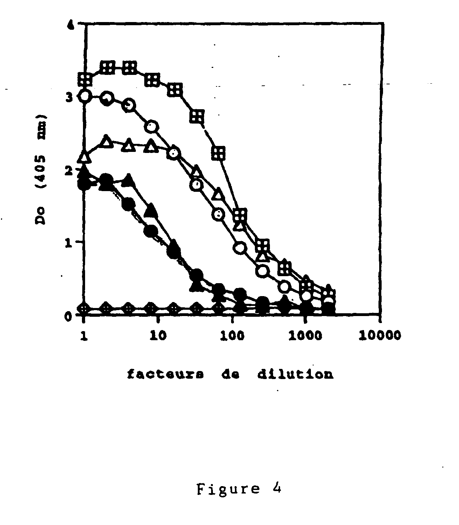

Les deux IgG sont ensuite révélées par un anticorps secondaire anti IgG couplé à la phosphatase alcaline. Le sérum biotynilé est révélé par de l'extravidine couplée à de la phosphatase alcaline. Les scFvs sont révélés par l'anticorps 9E10 anti-myc, puis par un anticorps anti IgG couplé à la phosphatase alcaline. Un dosage colorimétrique de l'activité phosphatase alcaline est représenté sur la figure 4, indiquant que les deux lgG purifiées, le sérum polyclonal et les ScFvs 421 et 11D3 reconnaissent p53 alors que le ScFv anti-CD3 est inactif.

### EXEMPLE 5 : Les ScFvs sont capables d'induire un supershift à la p53 sauvage

La capacité des ScFvs à activer la fonction DNA binding de la p53 sauvage a été testée par des experiences de retard sur gel.

Un duplex d'ADN représentant un site de fixation spécifique de p53 a été marqué au 32P puis incubé avec de la p53 sauvage purifiée et différents anticorps purifiés ou ScFvs produits dans des periplasmes bactériens. Les complexes sont résolus par électrophorèse sur gel d'acrylamide.

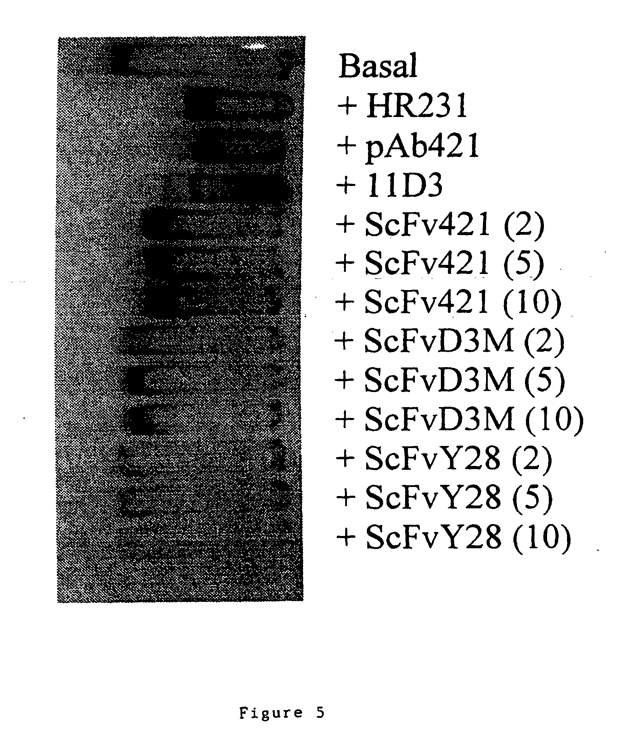

Les résultats obtenus sont présentés sur la Figure 5.

Le complexe ADN/p53 est observé dans la ligne « Basal ». Les anticorps HR231, pAb421 et 11D3 sont capables d'induire un retard suppléméntaire (supershift) et d'augmenter la quantité de complexe ADN/p53.

Les ScFvs 421 et D3M sont capables également d'induire un supershift alors qu'un ScFv controle anti-ras (Y28) est silencieux. Le ScFv421, contrairement au ScFv D3M induit une augmentation de la quantité de complexe p53/ADN.

### EXEMPLE 6 : Les scFvs sont capables de restaurer une fonction DNA binding à un mutant de p53

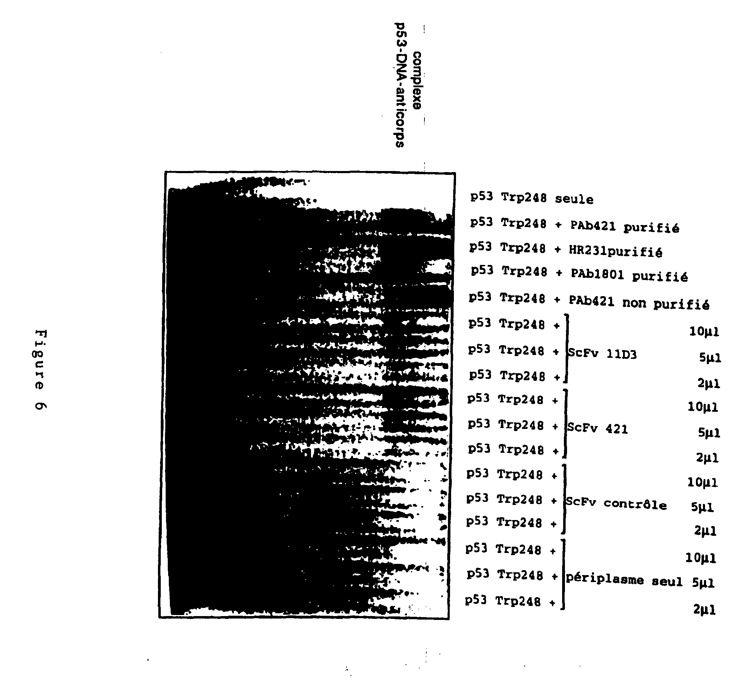

De manière analogue, Les ScFvs ont été téstés pour leur capacité à restaurer la fonction DNA binding du mutant Trp248 inactif. Les résultats obtenus sont présentés sur la Figure 6.

Ils montrent que les deux ScFvs induisent l'apparition d'une bande retardée correspondant à un complexe p53/ADN.

### EXEMPLE 7 : Les ScFvs sont correctement exprimés dans des cellules tumorales

L'expression des ScFvs a été vérifiée par transfection transitoire de vecteurs d'expression (promoteur de SV40) dans des cellules tumorales H1299. Plus particulièrement, les acides nucléiques ont été administrés sous forme d'un vecteur plasmidique de type pSV2 (exemple 3), en présence d'un lipide cationique, la lipofectamine.

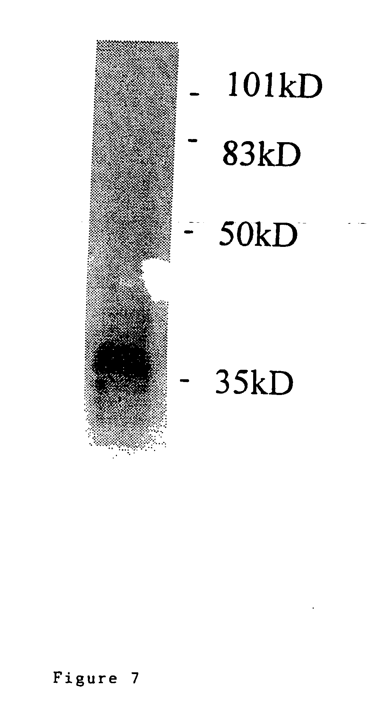

Les résultats obtenus sont présentés sur la Figure 7. Par Western blot sur un extrait total, on observe une bande majoritaire migrant vers les 30kD, qui correspond à la taille attendue. et confirme que les molécules sont exprimées à des niveaux significatifs dans les cellules tumorales.

### EXEMPLE 8 : Les ScFvs sont capables de restaurer partiellement la fonction transactivatrice des mutants His273.

La fonctionnalité de ces ScFvs au sein de cellules tumorales sur la fonction transactivatrice déficiente des formes mutées de p53 a été mesurée de la façon suivante:

Des transfections transitoires ont été réalisées dans des lignées H358 ou H1299 (toutes deux délétées pour p53) ou dans la lignée HT29 (comportant le mutant p53 His 273). Ces transfections introduisaient des vecteurs d'expression pour la p53 de type sauvage ou les mutants H273 ou His175, pour les deux ScFvs et un plasmide rapporteur comprenant le gene CAT (chloramphenicol acetyl transferase) sous le controle d'un promoteur dépendant de p53. L'activité CAT mesurée 48h après transfection est le reflet de la fonction transactivatrice de p53.

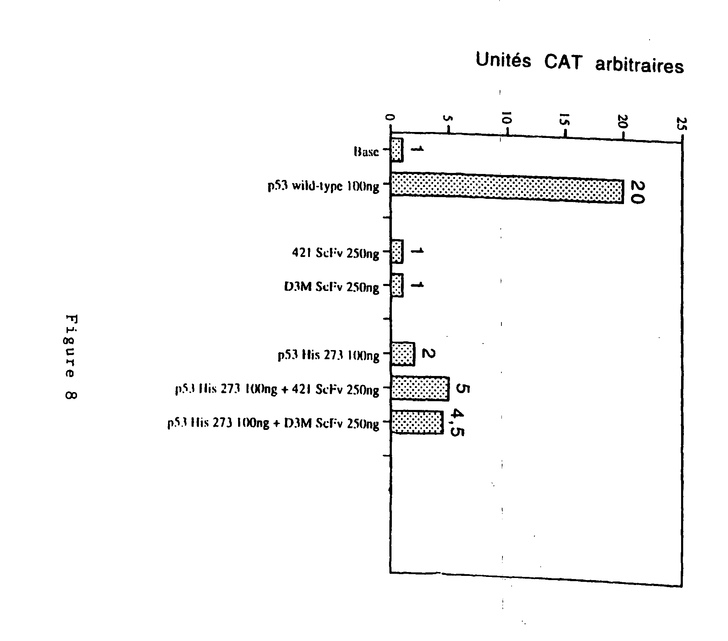

Les resultats de la figure 8 indiquent que dans la lignée H358, les deux ScFvs sont capables de réactiver de façon significative l'activité transcriptionnelle du mutant His273. des résultats identiques ont été obtenus dans la lignée H 1299.

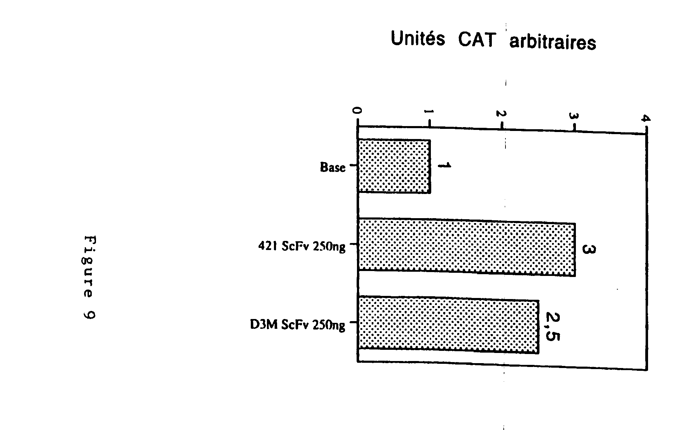

De même, dans la lignée HT29, les deux ScFvs sont capables d'augmenter l'activité transcriptionelle du mutant His273 endogène (Figure 9).

### SEQUENCES

SEQ ID n° 1 : Séquences nucléotidique et peptidique du 421

1/1 31/11
CAG GTG CAG CTG CAG CAG TCT GGG GCA GAG CTT GTG AGG TCA GGG GCC TCA GTC AAG TTG
gln val gln leu gln gln ser gly ala glu leu val arg ser gly ala ser val lys leu
61/21 91/31
TCC TGC ACA GCT TCT GGC TTC AAC ATT AAA GAC TAC TAT ATG CAC TGG GTG AAG CAG AGG
ser cys thr ala ser gly phe asn ile lys asp tyr tyr met his trp val lys gln arg
121/41 151/51
CCT GAA CAG GGC CTG GAG TGG ATT GGA TGG ATT GAT CCT GAG AAT GGT GAT ACT GAA TAT
pro glu gln gly leu glu trp ile gly trp ile asp pro glu asn gly asp thr glu tyr
181/61 211/71
GCC CCG AAG TTC CAG GGC AAG GCC ACT ATG ACT GCA GAC ACA TCC TCC AAT ACA GCC TAC
ala pro lys phe gln gly lys ala thr met thr ala asp thr ser ser asn thr ala tyr
241/81 271/91
CTG CAG CTC AGC AGC CTG GCA TCT GAG GAC ACT GCC GTC TAT TAT TGT AAT TTT TAC GGG
leu gln leu ser ser leu ala ser glu asp thr ala val tyr tyr cys asn phe tyr gly
301/101 331/111
GAT GCT TTG GAC TAT TGG GGC CAA GGG ACC ACG GTC ACC GTC TCC TCA GGT GGA GGC GGT
asp ala leu asp tyr trp gly gln gly thr thr val thr val ser ser gly gly gly gly
361/121 391/131
TCA GGC GGA GGT GGC TCT GGC GGT GGC GGA TCG GAT GTT TTG ATG ACC CAA ACT CCA CTC
ser gly gly gly gly ser gly gly gly gly ser asp val leu met thr gln thr pro leu
421/141 451/151
ACT TTG TCG GTT ACC ATT GGA CAA CCA GCC TCC ATC TCT TGC AAG TCA AGT CAG AGC CTC
thr leu ser val thr ile gly gln pro ala ser ile ser cys lys ser ser gln ser leu
481/161 511/171
TTG GAT AGT GAT GGA AAG ACA TAT TTG AAT TGG TTG TTA CAG AGG CCA GGC CAG TCT CCA
leu asp ser asp gly lys thr tyr leu asn trp leu leu gln arg pro gly gln ser pro
541/181 571/191
AAG CGC CTA ATC TAT CTG GTG TCT AAA CTG GAC TCT GGA GTC CCT GAC AGG TTC ACT GGC
lys arg leu ile tyr leu val ser lys leu asp ser gly val pro asp arg phe thr gly
601/201 631/211
AGT GGA TCA GGG ACA GAT TTC ACA CTG AAA ATC AAC AGA GTG GAG GCT GAG GAT TTG GGA
ser gly ser gly thr asp phe thr leu lys ile asn arg val glu ala glu asp leu gly
661/221 691/231
GTT TAT TAT TGC TGG CAA GGT ACA CAT TCT CCG CTC ACG TTC GGT GCT GGC ACC AAG CTG
val tyr tyr cys trp gln gly thr his ser pro leu thr phe gly ala gly thr lys leu
721/241
GAA ATC AAA
glu ile lys

SEQ ID n° 2 : Séquences nucléotidique et peptidique du D3M

1/1                                                    31/11
CAG GTC AAG CTG CAG GAG TCA GGG GCA GAA CTT GTG AGG TCA GGG GCC TCA GTC AAT TTG
gln val lys leu gln glu ser gly ala glu leu val arg ser gly ala ser val asn leu
61/21                                                  91/31
TCC TGC ACA GCT TCT GGC TTC AAC ATT AAA GAC TAC TAT ATG CAC TGG GTG AAA CAG AGG
ser cys thr ala ser gly phe asn ile lys asp tyr tyr met his trp val lys gln arg
121/41                                                 151/51
CCT GAA GAG GGC CTG GAG TGG ATT GGA TAT ATT GAT CCT GAG AGT GGT GAA ACT GAA TAT
pro glu glu gly leu glu trp ile gly tyr ile asp pro glu ser gly glu thr glu tyr
181/61                                                 211/71
GCC CCG AAC TTC CAG GGC AAG GCC ACT GTG ACT GCA GAC ACA TCC TCC AAC ACA GCC TAC
ala pro asn phe gln gly lys ala thr val thr ala asp thr ser ser asn thr ala tyr
241/81                                                 271/91
CTG CAC CTC AGC AGC CTG ACA TCT GAG GAC ACA ACC GTC TAT TAC TGT AAT GCA GTC ATC
leu his leu ser ser leu thr ser glu asp thr thr val tyr tyr cys asn ala val ile
301/101                                                331/111
TAC TAT GAA TAC GAC GGC TAT GCT TTG GAC TAC TGG GGC CAA GGG ACC ACG GTC ACC GTC
tyr tyr glu tyr asp gly tyr ala leu asp tyr trp gly gln gly thr thr val thr val
361/121                                                391/131
TCC TCA GGT GGA GGC GGT TCA GGC GGA GGT GGC TCT GGC GGT GGC GGA TCG GAC ATT GAG
ser ser gly gly gly gly ser gly gly gly gly ser gly gly gly gly ser asp ile glu
421/141                                                451/151
CTC ACC CAG TCT CCA TCT TCC CTG GCT GTG TCA GCA GGA GAG AAG GTC GCT ATG AGC TGC
leu thr gln ser pro ser ser leu ala val ser ala gly glu lys val ala met ser cys
481/161                                                511/171
AAA TCC AGT CAG AGT CTG TTC AAC AGT AGA ACC CGA AAG AAT TAC TTG GCT TGG TAT CAG
lys ser ser gln ser leu phe asn ser arg thr arg lys asn tyr leu ala trp tyr gln
541/181                                                571/191
CAG AAA CCA GGG CAG TCT CCT AAA GTG CTG ATC TAC TGG GCA TCC ACT AGG GAA TCT GGA
gln lys pro gly gln ser pro lys val leu ile tyr trp ala ser thr arg glu ser gly
601/201                                                631/211
GTC CCT GAT CGC TTC ACA GGC AGT GGA TCT GGG ACA GAT TTC ACT CTC ACC ATC AGC AGT
val pro asp arg phe thr gly ser gly ser gly thr asp phe thr leu thr ile ser ser
661/221                                                691/231
GTG CAG GCT GAA GAC CTG GCA GTT TAT TAC TGC AAG CAA TCT TAT AAT CTA CCG ACG TTC
val gln ala glu asp leu ala val tyr tyr cys lys gln ser tyr asn leu pro thr phe
721/241
GGC GGG GGC ACC AAG CTG GAA ATC AAA
gly gly gly thr lys leu glu ile lys

## Revendications

1. Utilisation d'un anticorps simple chaine capable de lier spécifiquement un épitope présent dans la région C-terminale de p53 comprise entre les résidus 320-393 pour la préparation d'une composition pharmaceutique destinée au traitement des cancers dans lesquels une protéine p53 mutée est impliquée.

2. Utilisation selon la revendication 1, **caractérisée en ce que** l'anticorps simple chaine est choisi parmi le ScFv421 de séquence SEQ ID N° 1 et le 11D3 de séquence SEQ ID N° 2.

3. Utilisation d'un acide nucléique codant pour un anticorps simple chaine capable de lier spécifiquement un épitope présent dans la région C-terminale de p53 comprise entre les résidus 320-393 pour la préparation d'une composition pharmaceutique destinée au traitement des cancers dans lesquels une protéine p53 mutée est impliquée.

4. Utilisation selon la revendication 3, **caractérisée en ce que** l'acide nucléique code pour un anticorps simple chaine choisi parmi le ScFv421 de séquence SEQ ID N° 1 et le 11D3 de séquence SEQ ID N° 2.

5. Utilisation selon la revendication 4, **caractérisée en ce que** l'acide nucléique fait partie d'un vecteur.

6. Utilisation selon la revendication 5, **caractérisée en ce que** le vecteur est un vecteur viral.

7. Utilisation selon la revendication 6, **caractérisée en ce que** le vecteur est un adénovirus recombinant défectif.

8. Utilisation selon la revendication 6, **caractérisée en ce que** le vecteur est un rétrovirus recombinant défectif.

9. Utilisation selon la revendication 6, **caractérisée en ce que** le vecteur est un AAV recombinant défectif.

10. Utilisation selon la revendication 6, **caractérisée en ce que** le vecteur est un HSV recombinant défectif.

11. Utilisation selon l'une des revendications 1 à 10, **caractérisée en ce que** le cancer est un cancer du poumon, du colon, tête et cou, hépatique, du cerveau.

12. Utilisation selon l'une des revendications 1 ou 3, **caractérisée en ce que** la protéine p53 mutée est choisie parmi les protéines p53H273, p53W248 et p53G281.

13. La molécule 11D3 telle que représentée dans la séquence SEQ ID N°2.

14. Acide nucléique codant pour une molécule selon la revendication 13.

15. Acide nucléique selon la revendication 14 **caractérisé par** la séquence SEQ ID N° 2.

16. Composition comprenant un acide nucléique selon la revendication 15.

17. Composition comprenant une molécule selon la revendication 13.

18. Composition pharmaceutique comprenant un acide nucléique selon la revendication 14 et un véhicule pharmaceutiquement acceptable, pour le traitement des cancers.

## Claims

1. Use of a single-chain antibody which is able to specifically bind an epitope present in the C-terminal region of p53 between residues 320-393, for preparing a pharmaceutical composition which is intended for treating cancers in which a mutated p53 protein is involved.

2. Use according to Claim 1, **characterized in that** the single-chain antibody is selected from ScFv421, having the sequence SEQ ID No. 1, and 11D3, having the sequence SEQ ID No. 2.

3. Use of a nucleic acid encoding a single-chain antibody which is able to specifically bind an epitope present in the C-terminal region of p53 between residues 320-393, for preparing a pharmaceutical composition which is intended for treating cancers in which a mutated p53 protein is involved.

4. Use according to Claim 3, **characterized in that** the nucleic acid encodes a single-chain antibody selected from ScFv421, having the sequence SEQ ID No. 1, and 11D3, having the sequence SEQ ID No. 2.

5. Use according to Claim 4, **characterized in that** the nucleic acid is part of a vector.

6. Use according to Claim 5, **characterized in that** the vector is a viral vector.

7. Use according to Claim 6, **characterized in that** the vector is a defective recombinant adenovirus.

8. Use according to Claim 6, **characterized in that** the vector is a defective recombinant retrovirus.

9. Use according to Claim 6, **characterized in that** the vector is a defective recombinant AAV.

10. Use according to Claim 6, **characterized in that** the vector is a defective recombinant HSV.

11. Use according to one of Claims 1 to 10, **characterized in that** the cancer is a lung cancer, a colon cancer, a head and neck cancer, a liver cancer or a brain cancer.

12. Use according to either of Claims 1 and 3, **characterized in that** the mutated p53 protein is selected from the proteins p53H273, p53W248 and p53G281.

13. The 11D3 molecule as represented in the sequence SEQ ID No. 2.

14. Nucleic acid encoding a molecule according to Claim 13.

15. Nucleic acid according to Claim 14, **characterized by** the sequence SEQ ID No. 2.

16. Composition comprising a nucleic acid according to Claim 15.

17. Composition comprising a molecule according to Claim 13.

18. Pharmaceutical composition comprising a nucleic acid according to Claim 14 and a pharmaceutically acceptable carrier, for treating cancers.

## Patentansprüche

1. Verwendung eines einkettigen Antikörpers, der spezifisch an ein Epitop in der C-terminalen Region von p53 zwischen den Resten 320-393 binden kann, zur Herstellung einer pharmazeutischen Zusammensetzung, bestimmt zur Behandlung von Krebserkrankungen, bei denen ein mutiertes p53-Protein beteiligt ist.

2. Verwendung nach Anspruch 1, **dadurch gekennzeichnet, dass** der einkettige Antikörper aus ScFv421 der Sequenz SEQ ID NR: 1 und 11D3 der Sequenz SEQ ID NR: 2 ausgewählt ist.

3. Verwendung einer Nukleinsäure, die für einen einkettigen Antikörper codiert, der spezifisch an einen Epitop in der C-terminalen Region von p53 zwischen den Resten 320-393 binden kann, zur Herstellung einer pharmazeutischen Zusammensetzung, bestimmt zur Behandlung von Krebserkrankungen, bei denen ein mutiertes p53-Protein beteiligt ist.

4. Verwendung nach Anspruch 3, **dadurch gekennzeichnet, dass** die Nukleinsäure für einen einkettigen Antikörper codiert, der aus ScFv421 der Sequenz SEQ ID NR: 1 und 11D3 der Sequenz SEQ ID NR: 2 ausgewählt ist.

5. Verwendung nach Anspruch 4, **dadurch gekennzeichnet, dass** die Nukleinsäure Teil eines Vektors ist.

6. Verwendung nach Anspruch 5, **dadurch gekennzeichnet, dass** der Vektor ein viraler Vektor ist.

7. Verwendung nach Anspruch 6, **dadurch gekennzeichnet, dass** der Vektor ein defektives rekombinantes Adenovirus ist.

8. Verwendung nach Anspruch 6, **dadurch gekennzeichnet, dass** der Vektor ein defektives rekombinantes Retrovirus ist.

9. Verwendung nach Anspruch 6, **dadurch gekennzeichnet, dass** der Vektor ein defektives rekombinantes AAV ist.

10. Verwendung nach Anspruch 6, **dadurch gekennzeichnet, dass** der Vektor ein defektives rekombinantes HSV ist.

11. Verwendung nach einem der Ansprüche 1 bis 10, **dadurch gekennzeichnet, dass** die Krebserkrankung ein Lungen-, Enddarm-, Kopf-Hals-, Leber-, Hirnkrebs ist.

12. Verwendung nach einem der Ansprüche 1 bis 3, **dadurch gekennzeichnet, dass** das mutierte p53-Protein aus den Proteinen p53H273, p53W248 und p53G281 ausgewählt ist.

13. Das Molekül 11D3, wie in der Sequenz SEQ ID NR: 2 dargestellt.

14. Nukleinsäure, die für ein Molekül nach Anspruch 13 codiert.

15. Nukleinsäure nach Anspruch 14, **gekennzeichnet durch** die Sequenz SEQ ID NR: 2.

16. Zusammensetzung, umfassend eine Nukleinsäure nach Anspruch 15.

17. Zusammensetzung, umfassend ein Molekül nach Anspruch 13.

18. Pharmazeutische Zusammensetzung, umfassend eine Nukleinsäure nach Anspruch 14 und ein pharmazeutisch annehmbares Vehikel zur Behandlung von Krebserkrankungen.
